# EUROPEAN PATENT SPECIFICATION

(11) **EP 2 825 020 B1**
(45) Date of publication and mention of the grant of the patent: **04.05.2016**
(21) Application number: 13707398.7
(22) Date of filing: 05.03.2013
(51) Int. Cl.: A01G 9/10, A01H 4/00

(54) **CONTAINER FOR STORING AND PLANTING SEEDS, BULBS OR TUBERS**
BEHÄLTER ZUM LAGERN UND AUSSÄEN VON SAMEN, ZWIEBELN ODER KNOLLEN
RÉCIPIENT POUR CONSERVER ET PLANTER DES GRAINES, DES BULBES OU DES TUBERCULES

(30) Priority: 16.03.2012 LU 91959
(43) Date of publication of application: 21.01.2015
(73) Proprietor: Luxembourg Institute of Science and Technology (LIST), 4362 Esch-sur-Alzette (LU)
(72) Inventor: SCHLEEH, Thomas, 54338 Schweich (DE); HAUSMAN, Jean-François, B-6750 Musson (BE)
(74) Representative: Lecomte & Partners
(86) International application number: PCT/EP2013/054397
(87) International publication number: WO 2013/135530

(56) References cited:
- US-A- 4 769 945
- US-A- 5 427 593
- US-A1- 2009 005 245

## Description

### Field of the invention

The present invention is directed to a planting container or box, in particular to a container for storing and planting seeds, bulbs, or tubers.

### Background of the invention

Many systems and methods for planting seeds or seedlings are known in the prior art.

Normally plants are raised in a nursery garden or in a greenhouse up to a desired size and are then replanted to a habitat. However, until the plantlings have reached that desired size they usually require constant care. In particular, they must be provided with soil and water. Further, such plantlings may occupy a considerable space, which is provided in many cases in a building in order to protect the plantlings from chilling or freezing, drought, strong winds or too intensive sun light.

Another disadvantage is that the sensitive plantlets must be handled with care on their way from a nursery garden to their destination. Depending on the time needed for transport of the plantlets to their final habitat, they require water or sun light to survive. After arrival at the planting position, usually a hole has to be dug and an additional amount of water is required for ensuring growth of the plant at that position. Although the amount of water required might be small, it has to be ensured that water is applied exactly at the position of the plantlet.

Further issues arise when planting large amounts of plants. In case of forestation, hundreds or even thousands of plants or trees have to be provided. Further, the areas for forestation are often difficult to access. Thus, also for this reason the costs for planting may become unsatisfactorily high. Further problems may arise if the ground envisaged for planting is very hard, stony, dry or comprises already a large amount of roots.

A plant container is known from document US-A-2009/005245.

US patent 3,273,284 discloses a planting container for planting seedlings by way of an air-borne drop. The planting container comprises a seedling provided in a wire mesh provided in an elongate receptacle made of a material which disintegrates upon exposure to water. A water-filled bladder is provided below the wire mesh containing the seedling, which is pierced by a needle upon impact of the planting container on the ground. The water spilled from the pierced bladder degrades immediately the receptacle and allows the seedling to enroot in the surrounding soil.

However, that planting container may especially be ineffective in case of very hard, stony frozen or already enrooted ground since it may not penetrate into such a surface. Another disadvantage of the mentioned disclosure consists in the fact that the plant gets into contact with the environment directly after its drop. This may result in death of the plantling if it is dropped in a hostile, e.g. polluted area. Further, the known planting container is difficult and expensive to produce and assemble. In particular, the seedling has to be raised first and is then included in the container, thus complicating also the transport of that device. Convenient and cheap long-term storage of the container is not possible since the plantlet would die without sufficient water supply or exposure to sunlight.

Another general issue consists in creating new habitats in barren, extremely dry or polluted areas. In such cases experience has shown that plantlets or seedlings often fade after some time of growth. Reasons might be a lack of water or nutrients or an amount of hazardous substances unbearable for the young plant before having reached a certain crucial size.

The technical problem of the present invention is to provide an advanced, preferably compact, planting container or device enabling storage and/or planting of seeds, bulbs or tubers.

In particular, the device shall enable an easy and/or cheap long-term storage of a planting container and/or an easy and undemanding transportability.

Further, the planting container should be suitable for planting in areas difficult to access and independent of the hardness of the ground in such areas.

In particular, the growing plant should be protected from strong winds, and preferably from too intensive sunlight.

In particular, the plant should also be protected from hazardous substances in polluted areas for a determinable time.

Another object of the invention may consist in overcoming at least one of the above mentioned disadvantages.

### Summary of the invention

The above mentioned technical problem is solved by the subject-matter of appended claim 1 which is directed to a (closed / autarkic / self-sufficient) container (or box / receptacle) for storing and planting seeds, bulbs, or tubers. The container comprises a housing made of a non-water soluble material, the housing comprising a first compartment for receiving at least one seed, bulb or tuber, the first compartment being essentially free of water (and preferably also free of nutrients), and a second compartment for receiving nutrients promoting growth of the seed, bulb or tuber, the second compartment being arranged below the first compartment. In accordance with the present invention the first and the second compartments are separated by a separation layer. Preferably, the housing has a rounded (in particular an essentially half-spherical) shape at its bottom, and the container has a center of gravity arranged such that the container is adapted to erect itself (to stand automatically upright) when it is deposited on a supporting surface in a tilted manner.

The container according to the present invention provides several advantages for storing and plant growth:

For example, providing a non-water soluble housing ensures that the plant may germinate or start growing without contact to the environment. This might for example be of advantage in polluted areas in which the plant needs to reach a certain critical size in order to be viable under such negative conditions.

Further, the container may even be used in areas with a very hard, dry, frozen or already enrooted ground in which it is difficult or nearly impossible to entrench a plant. Also this feature helps to avoid a premature contact of the seed or plant with the environment. At the same time an automatic upright standing ensures optimal conditions for the plant's growth. In particular, this feature is of advantage in case of an aerial or air-borne drop. The shape of the housing could for example be essentially spherical. However, other shapes are also within the scope of the present invention as for example essentially half-spherical, ellipsoidal or drop-like shapes.

It is remarked that the container is not intended to be used with or to comprise a seedling, i.e. a small plant germinated already. Such a seedling usually needs water, sunlight and/or sufficient temperatures to stay alive. Such aspects would result in heavy restrictions and high costs with respect to long-term storage. According to the present invention, the seed, bulb or tuber is separated from any water when lying in the first compartment. Since the upper and the lower compartments are separated from each other by the separation layer and the upper compartment does not contain water, a long-term storage of the system may be possible.

Further, it is assured that the second compartment is below the first compartment after deposition so that the seed, tuber or bulb is positioned above the nutrients. The capability of a self-orientation of the container may also be crucial for further preferred aspects of the invention described below.

The present invention can also be directed to a container for storing and planting seeds, bulbs or tubers, wherein the container comprises a housing made of a non-water soluble material, the housing comprising a first compartment for receiving at least one seed, bulb or tuber, the first compartment being essentially free of water (and preferably also free of nutrients), and a second compartment for receiving nutrients promoting growth of the seed, bulb or tuber, the second compartment being arranged below the first compartment, wherein the first and the second compartments are separated by a separation layer and wherein the container comprises (activation) means or an activation system for destruction of the separation layer.

According to an aspect of the present invention, the second compartment comprises a (firm or viscous) nutrient matrix, preferably a gel for storing water. Such a nutrient matrix allows long-term storage of the container and can comprise any nutrition the plant will need for a certain time of growth. The composition of the nutrient matrix can for example be a plant nutrient solution stabilized in a gelling agent like agar, pectin, glutamate, or any other solidifier. Of course such nutrition can be adapted by the person skilled in the art to the used sort of plant. Thus, the container may be even deposited in barren areas, wherein especially at the beginning of the plant's growth, the plant is very sensitive to sufficient water supply and appropriate nutrition. After some time of growth the plant may be strong or tall enough to grow further on the basis of the soil surrounding the deposited container. In general, nutrients may be already present in the container during storage or could be added later by a customer. For example, the housing could include a screw closure for opening the container between the separation layer and the bottom of the container so that nutrients could be applied to the second compartment by a customer. In general, one nutrient composition could be used for several seeds. Another possible advantage consists in that the nutrient composition can e.g. be chosen for a seed on a short term according to current environmental conditions. Moreover, the geometry could be selected on a short term according to the landscape where it will be used. Alternatively or in addition, the housing could comprise any other kind of closure or gate allowing introduction of nutrients into the lower compartment. Alternatively or in addition, the same could apply to placement of a seed, bulb, tuber or seedling. In particular, the housing or the first compartment may comprise a screw closure for placing a seed, tuber, bulb or seedling into the first compartment. Alternatively or in addition, the first compartment may comprise any other kind of closure or gate allowing introduction of a seed, bulb or tuber. Screw closures might for example be circumferential with the housing (preferably essentially parallel to the plane of the separation layer). Alternatively a screw closure might be provided in the form of screw cap allowing access to one or both of the compartments. As another alternative, one or both of the compartments could be provided with a watertight plug, which can be opened to equip the respective compartments with a seed, bulb, tuber or nutrients, respectively.

According to another aspect of the invention the housing comprises biodegradable materials or portions with reduced wall thickness or combinations thereof. Thus, after a desired time the housing may degrade so that the plant may get into contact with the environment and the ground surrounding the device. Thus, roots start to enroot into the surface of soil beside or below the device. Further, the wall thickness and/or the material of the housing may be designed such that the plant may penetrate the housing after a certain time of growth within the container.

Thus, according to another aspect of the invention the housing is penetrable by roots or is destroyable by roots originating from the seed, bulb or tuber. Preferably, the housing comprises one or more of the following materials: paper, carton, plastic, rubber, ceramics, glass, pottery, layer of salt, sheets of metal, textiles or braids of such materials. The thickness and exact material may be chosen by the person skilled in the art and may depend on the plant to be planted.

According to yet another aspect of the invention, the container comprises (activation) means for destruction of the separation layer. For example, such means could comprise means for mechanically penetrating and/or breaking the separation layer, as e.g. a needle or object for penetrating the separation layer adapted to be manually pressed from outside of the container through the separation layer. As another example, the housing might comprise at least one flexible portion adjacent the separation layer so that the separation layer may be destroyed by pressing the flexible portion. Such manual means for destructing the separation layer or in other words for activating the container or trigger germination or growth of a seed, tuber or bulb can be easily and quickly handled for a large number of devices without any need of specially trained or educated personnel. In particular, if the housing is made of one of paper, carton, plastic, sheets of metal it may be adapted to be flexible for destruction of the separation layer by exerting a manual force on the housing.

Other means for activation could comprise a chemical dissolution of the separation layer. For example, the separation lay could be partially made of water soluble-salts. The container could comprise a pocket of water destructible by mechanical pressure. Thus, such a pocket could be destroyed by mechanical means as mentioned above or for example by pressure on a flexible portion of the device's housing. Of course the described dissolution is not limited to the application of water-soluble salts. Other combinations of chemicals could be used as well.

As another example, the separation layer is made of a material melting or degrading above a defined temperature. Such a temperature could be for example between 20 °C and 50 °C, preferably between 25 °C and 45 °C. Thus, the separation layer could comprise or be made of a wax which melts after the container has been heated by sunlight. However, the separation layer is preferably essentially water-free.

According to still another aspect of the invention, the separation layer is a brittle plate, grid, braid or a film, e.g. made of salts, ceramics, glass, plastics, etc. The separation layer could also be a sheet of paper or card board. In particular, the separation layer has a planar shape extending essential in a horizontal direction. Alternatively or in addition the separation layer may comprise a depression for receiving the seed, bulb or tuber. Such a depression could be arranged at or near the center of the compartment so that the seed, bulb or tuber has better starting condition for growth. In particular, this feature may promote uniform growth and enrooting of the plant in the nutrients of the lower compartment.

According to yet another aspect of the invention, the housing is watertight with respect to the environment of the container at least in the portion of the second compartment. Alternatively, the housing is (completely) watertight with respect to the environment of the device. This feature improves the separation between the inner volume of the housing and the environment of the device. Thus, on the one hand, premature germination or growth during storage of the container is excluded and on the other hand, an early contact of the plant with the surrounding environment after deposition of the container to a planting location is avoided. Preferably, the housing is opaque.

According to yet another aspect of the invention, the housing has at least one aperture in a top area of the container, the aperture being covered by a cover layer (/ film or foil). Such a cover layer could be either opaque or transparent depending on the specific application or plant to be planted.

Preferably, the cover layer comprises an at least partially transparent foil or film for enabling a greenhouse effect within the housing. Thus, the plant may enjoy a greenhouse effect similar as under the conditions in a greenhouse or a plant production facility.

Preferably, the cover foil is penetrable for a plant originating from the seed, bulb, tuber or seedling. In other words, the growing plant may pierce or break the cover foil during growth. Then rain may fall through such an opening into the device. Thus, the young plant will be supplied by additional water but may be still without contact to soil surrounding the container.

According to another aspect of the present invention, the first compartment comprises an additional cover layer (/ foil or film) arranged between a cover layer covering the aperture and the separation layer. Thus, the separation layer may comprise a transparent foil and the additional cover foil might be for instance an opaque layer. Alternatively, the cover foil covering the aperture is opaque and the cover foil between the latter and the separation layer comprises a transparent foil. An opaque layer may be of desire in case of a plant which shall germinate in darkness. The additional cover foil allows for a greenhouse effect.

In general, the application of the above mentioned foils or films constitutes an easy and cheap manner of providing containers with considerable abilities.

According to another aspect of the invention, at least one of the cover layers (or both) comprises a slit or hole for introducing a seed, tuber, bulb into the first compartment. Thus, the container could for example be sold or sent to customers without a seed, tuber or bulb and be self-equipped or filled by the customer. Alternatively, the first compartment may comprise a seed, bulb, tuber or seedling by default.

According to still another aspect of the invention the separation layer comprises nutrition, preferably in the form of an inorganic salt as for example Hydroxyapatite, Ca₃(PO₄)₂, KCl, CaNaPO₄ * CaSiO₄, (NH₄)₂HPO₄. Thus, the present container offers a further possibility of providing further substances for promoting the plant's growth inside the device.

The present invention is also directed to a method of using the above mentioned device. In particular, the container according to the present invention may be stored in a first step and may be activate in a subsequent step by destruction of the separation layer such that the seed, bulb or tuber gets into contact with the nutrient matrix in the lower compartment. Afterwards, the container is deposited onto a surface. The surface could for example be an area of land or soil. Alternatively, it could be a water surface or also the ground of a lake, a pond or the sea.

All features of the above described aspects may be combined or replaced with one another.

### Brief description of the drawings

In the following, we briefly describe the figures according to embodiments of the present invention. Further details are given in the detailed description of the embodiments. The figures have the purpose of illustrating the invention and should not be understood in a limiting sense.
- Figure 1: depicts a schematic perspective view of an embodiment of a container according to the present invention;
- Figure 2: depicts a schematic perspective view of another embodiment of a container according to the present invention, wherein the container comprises separation walls extending essentially perpendicular to the separation layer;
- Figure 3: depicts a schematic perspective view of another embodiment of a container according to the present invention, comprising mechanical activation means for destruction of the separation layer;
- Figure 4: depicts a schematic perspective view of another embodiment of a container according to the present invention comprising a further cover layer above the separation layer; and
- Figure 5: depicts a schematic perspective view of another embodiment of a container according to the present invention.

### Detailed description of the embodiments

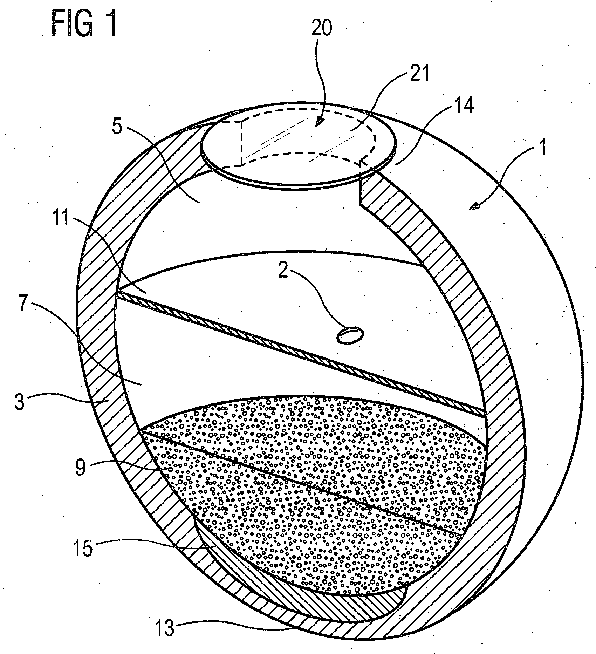

Figure 1 depicts one exemplary embodiment of a container 1 according to the present invention. The container comprises a housing 3 depicted with an essentially spherical shape. The housing 3 is divided into a first 5 and a second 7 compartment by a separation layer 11. The separation layer 11 has preferably a planar shape and may extend essentially in a horizontal direction. The first compartment 5 may comprise a seed 2, wherein for the sake of conciseness, the word seed 2 is used in the following as a synonym to a bulb 2 or a tuber 2. The second compartment 7 is arranged below the first compartment 5 or in other words below the upper compartment 5. The lower compartment 7 may receive or comprise nutrients 9. Such nutrients may be provided in the form of a nutrient matrix or in the form of a gel. Preferably, the lower compartment 7 comprises also water. Water may be bound in a matrix or a gel. According to the depicted embodiment, the container 1 (or the housing 3) comprises a mass or a weight 15. Such a mass 15 could e.g. be made of metal, stone, ceramics, etc. It could have different shapes not restricted to the depicted plate-like form. More compact shapes as e.g. spherical or cubic shapes could be used as well. However, it is not necessary to use a separate mass 15 as depicted in Figure 1. It is also possible to design the housing 3 as such with an appropriate mass distribution so that together with the rounded shape of the housing's 3 bottom 13, the container 1 may be adapted to stand automatically upright. The exact design may also depend on the type, size and number of seeds 2 to be planted with the container 1 as well as on the amount of nutrition and the size of the housing 3. Anyhow these parameters can be chosen by the person skilled in the art depending on the desired application of the container 1. The container 1 has been depicted with an essentially spherical housing 3. However, other shapes are also possible, as for example half-spherical, drop-like, or ellipsoid shapes.

As also depicted in Figure 1, the housing may comprise an upper aperture or window 20 which may be covered by a layer or foil 21. In particular, an at least partially transparent foil 21 results in the effect that a greenhouse effect may be provided within the housing 3 for promoting germination of the seed 2 or growth of a respective plant. Alternatively, the foil 21 may be essentially opaque so that a-seed 2 may germinate in darkness. The foil 21 has preferably a planar shape and may extend essentially in a horizontal direction. Of course, the foil 21 or the opening 20 do not need to have a circular shape other shapes may also be possible. The foil 21 could for example be made of plastics or paper material. The same applies to the housing 3. However, in general the housing is made of a non-water soluble material in order to assure that the seed 2 may germinate or grow for a desired time without getting into contact with the environment outside the housing 1. Preferably, the housing material is biodegradable. In addition, the housing 3 can be penetrable by the roots of the plant resulting from the seed 2, so that the plant may enroot in the environment of the container 1.

In general, the container 1 may have a shape with a maximum dimension or diameter smaller than 30 cm, preferably smaller than 20 cm, or even more preferably smaller than 10 cm. The container 1 is free of any supply lines. In particular it does not need or have any external electrical current lines or water supply lines. Further, the container 1 does not have to be stored under specific conditions. In particular, freezing of the container 1 is not required for storage purposes. It is storable at room temperature. Consequently, the storage and use of the container 1 is simple and cheap and does not require costly infrastructure and equipment.

Preferably, the container 1 can be activated or triggered to start germination of the seed 2. Such activation is in general provided via a destruction of the separation layer 11, as e.g. by dissolution, piercing, breaking, etc. For example, the separation layer 11 could be made of a brittle material which breaks upon pressure (mechanical shock) on an adjacent (outer) area of the housing 3. For this purpose the housing could be at least partially deformable adjacent to the separation layer 11. If, for example, the housing is made of paper, plastics, or carton (card board) such deformability is provided. By applying a pressure to the housing, the separation layer 11 may be broken so that the seed 2 gets into contact with the ingredients of the lower compartment 7. In particular, the contact with water stored or bound in the lower compartment 7 may trigger the germination of the seed 2.

Before activation, the upper compartment 5 is essentially free of water in order to avoid a premature germination or growth during storage of the container 1. Preferably, the upper compartment is also free of nutrients and/or any soil.

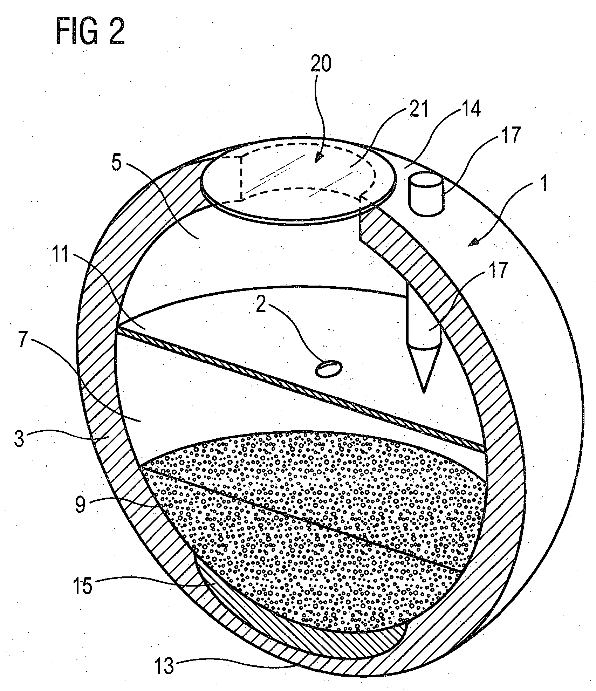

According to Figure 2, the container 1 is equipped with another kind of mechanical activation means 17 for destruction of the separation layer 11. The depicted container comprises a pin or a needle 17 for piercing or breaking the separation layer 11. Breaking the separation layer 11 allows a contact of the seed 2 with the nutrients and water stored in the lower compartment 7.

Alternatively, the activation means 17 could pierce a pocket of water or chemicals for dissolving the separation layer 11 (not shown). According to another alternative, the activation means could comprise an injection nozzle or syringe for introducing from outside the housing 3 water or chemicals into the housing 3 to dissolve the separation layer 11.

In general, activation means for destruction of the separation layer 11 can be integrated in the container 1 such that activation of the container 1 does not require any supply lines (as e.g. electrical current). The application of manual force may be sufficient.

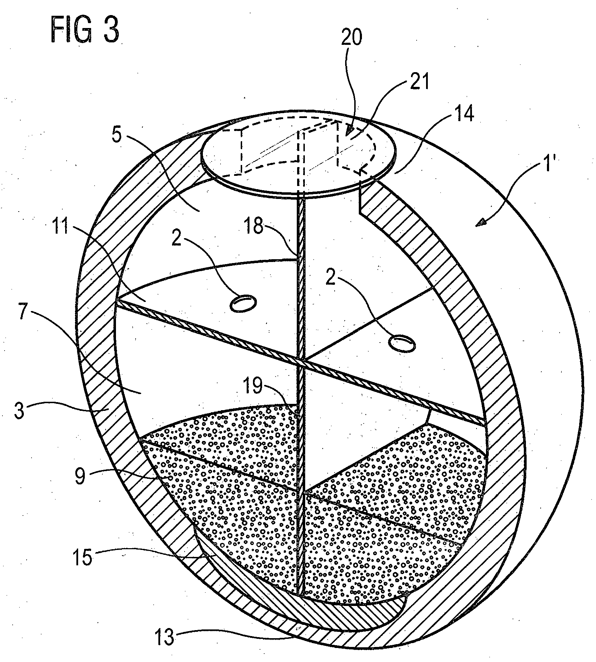

Figure 3 shows another embodiment of a container 1' in accordance with the present invention. In particular, this container 1 comprises two seeds 2 within separate chambers of the first compartment 5 divided by an essentially vertically extending separation wall 18. Each chamber of the first compartment 5 preferably comprises one seed 2. As depicted, it is possible that the lower compartment 7 is also divided by a second essentially vertically extending separation wall 19. Thus, each seed 2 may be provided with an own chamber in the first compartment 5 and with a corresponding chamber in the second compartment 7. The respective nutrition and water supply in the corresponding chamber of the second compartment 7 may be adapted to the requirements of the specific seed 2. The material of the separation wall may be non-biodegradable and/or not penetrable by the plant's roots in order to minimize competition between the plants growing the separate chambers. In particular, different kinds of seeds, bulbs or tubers 2 may be provided in one container 1'. Each of them may be provided with appropriate nutrients and water supply. The size of each chamber may be adapted to the specific size and sort of the seed, bulb, or tuber 2. As depicted, a common wall 18, 19 may run through both compartments 5, 7. The walls 18, 19 have been depicted as being essentially perpendicular to the separation layer 11. However, the walls may extend also in other directions. Preferably, the wall 18 of the upper compartment 5 extends from the separation layer 11 to a portion of the cover foil 21 such that this foil can be pierced by a plant growing in its chamber and/or such that light can still fall through a transparent cover foil 21 into each chamber of the first compartment 5.

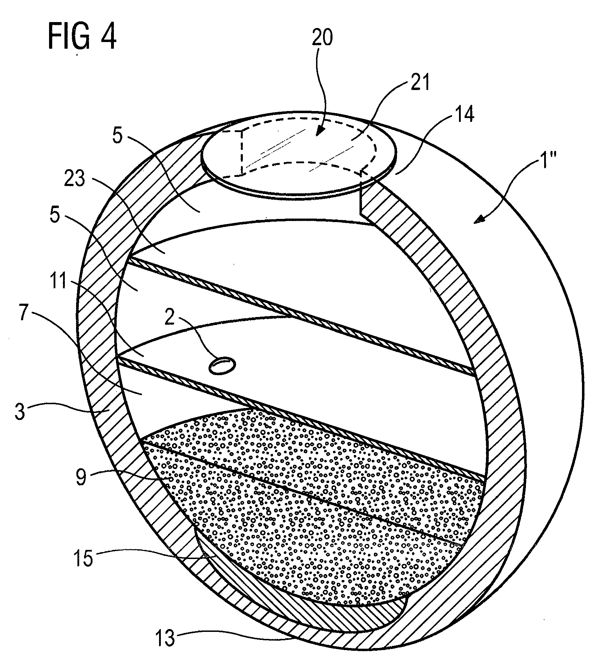

Figure 4 depicts a further container 1" in accordance with an embodiment of the present invention. In contrast to the container 1 as depicted in Figure 1, the container 1" comprises a second cover foil 23 arranged between a cover foil 21 covering the hole in a top portion 14 of the housing 3 and the separation layer 11. For example, the additional foil 23 could be essentially opaque or black and the cover foil 21 could be transparent. Such an arrangement allows generation of a greenhouse effect between the cover foil 21 and the additional cover foil 23. The heat will also be conducted to the remainder of the container 1". This may be of advantage for seeds 2 which germinate in darkness. After a certain time of growth plants 2 resulting from such seeds 2 may break the opaque cover layer 23 so that they may receive light shining through the transparent window 21. Preferably, that transparent cover foil 21 may also be broken by the further growing plant 2.

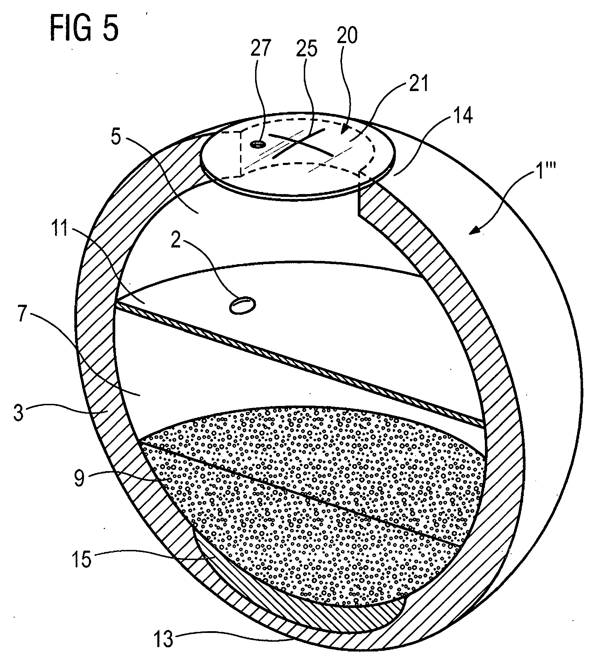

Figure 5 depicts another embodiment of a container 1"' according to the present invention. In contrast to the container 1 depicted in Figure 1, the cover foil 21 covering the aperture 20 in the top portion of the housing 3 comprises slits 25 and apertures 27. Slits 25 and/or apertures 27 may be used for introducing or placing a desired seed 2 into the upper compartment 5. In addition, or alternatively, such slits 25 or apertures 27 may be used to provide access for an activation means 17 (either mechanical or chemical) for destructing the separation layer 11. Slits 25 or apertures 27 may e.g. be cut by a knife. Alternatively, or in addition, slits 25 and/or apertures 27 may be present in the housing 27 so that seeds 2, tools or nutrients 9 may be introduced from outside the container into one of the compartments 5, 7.

The invention has been described with reference to best modes of carrying out the invention. Obviously, modifications and alterations will occur to others upon a reading and understanding of this specification. It is intended to include all such modifications and alterations in so far as they come within the scope of the appended claims or the equivalents thereof.

In any case the above described embodiments shall not be understood in a limiting sense. In particular, the features of the above embodiments may also be replaced or combined with one another.

### List of reference signs

- 1: container / planting box
- 1': container / planting box
- 1": container / planting box
- 1"': container / planting box
- 2: seed / bulb / tuber
- 3: housing / body housing
- 5: first compartment / upper compartment
- 7: second compartment / lower compartment
- 9: nutrients / nutrient matrix / gel
- 11: separation layer
- 13: bottom portion of the container / housing
- 14: top portion of the container / housing
- 15: weight
- 17: activation means for destruction of separation layer
- 18: separation wall
- 19: separation wall
- 20: aperture
- 21: cover layer / foil
- 22: separation wall / foil
- 23: additional cover layer / foil
- 25: slit
- 27: hole

## Claims

1. A container (1) for storing and planting seeds, bulbs or tubers (2), the container (1) comprising:
a housing (3) made of non-water soluble material, the housing comprising:
a first compartment (5) for receiving at least one seed, bulb or tuber (2), the first compartment (5) being essentially free of water,
a second compartment (7) for receiving nutrients (9) enabling growth of the seed, bulb or tuber (2), the second compartment (7) being arranged below the first compartment (5), and
a bottom portion (13); wherein the container (1) comprises further
a separation layer (11) separating the first and the second compartments (5, 7), and wherein the bottom portion (13) of the housing (3) has a rounded shape **characterised in that**, the container (1) has a center of gravity arranged such that the container (1) is adapted to erect itself when it is deposited on a supporting surface in a tilted manner.

2. The container according to claim 1, wherein the second compartment (7) comprises a nutrient matrix (9) comprising water, the water being preferably bound in the nutrient matrix (9) and/or wherein the nutrient matrix (9) is preferably a gel for storing water.

3. The container according to claim 1 or 2, wherein the housing (3) consists of non-water soluble, biodegradable materials and/or has portions with a reduced wall thickness.

4. The container according to one of the preceding claims, wherein the housing (3) is penetrable by roots originating from the seed, bulb or tuber (2), and wherein the housing (3) comprises preferably one or more of the following materials: paper, carton, plastics, sheets of metal or braids of those materials.

5. The container according to one of the preceding claims, wherein the container (1) comprises activation means (17) for destruction of the separation layer (11).

6. The container according to claim 5, wherein the activation means (17) comprises means for mechanically penetrating and/or breaking (17) the separation layer (11), or means for chemically dissolving the separation layer (11), or wherein the separation layer (11) is made of an essentially water-free material melting above a defined temperature.

7. The container according to one of the preceding claims, wherein the separation layer (11) is a, preferably brittle, plate, grid, braid or film.

8. The container according to one of the preceding claims, wherein the housing (3) is at least watertight with respect to the environment of the container (1) in a portion of the second compartment (7) or, wherein the housing (3) is watertight with respect to the environment surrounding the container (1).

9. The container according to one of the preceding claims, wherein the housing (3) has at least one aperture (20) in a top area (14) of the housing (3), the aperture (20) being covered by a preferably water-tight cover layer (21).

10. The container according to claim 9, wherein the cover layer (21) comprises an at least partially transparent foil for enabling a greenhouse effect within the housing (3), the cover layer (21) being preferably penetrable for a plant originating from the seed, bulb or tuber (2).

11. The container according to claim 9 or 10, wherein the first compartment (5) comprises an additional cover layer (23) arranged between the cover layer (21) covering the aperture (20) and the separation layer (11) such that a first space is provided between the additional cover layer (23) and the separation layer (11) and a second space is provided between the additional cover layer (23) and the cover layer (21) covering the aperture.

12. The container according to claim 11, wherein one of the cover layers (21, 23), preferably the additional cover layer (23), is essentially opaque.

13. The container according to one of claims 9 to 12, wherein at least one of the cover layers (21, 23) or the housing (3) comprises a slit (25) or a hole (27) for introducing a seed, tuber or bulb (2) into the first compartment (5); or wherein the first compartment (5) comprises a seed, bulb or tuber (2).

14. The container according to one of the preceding claims, wherein the separation layer (11) comprises nutrition for the seed, bulb or tuber (2), preferably in the form of an inorganic salt, in particular Hydroxyapatite, Ca₃(PO₄)₂ or KCI.

15. A method of using the container (1) according to one of the preceding claims, the method comprising the sequential steps of:
a) storing the container (1);
b) activating the container (1) by destructing the separation layer (11); and
c) depositing the container (1) on a surface envisaged for planting the seed, bulb or tuber (2).

## Patentansprüche

1. Behälter (1) zum Lagern und Pflanzen von Samen, Zwiebeln oder Knollen (2), wobei der Behälter (1) umfasst:
ein aus nicht wasserlöslichem Material hergestelltes Gehäuse (3), wobei das Gehäuse umfasst.
ein erstes Fach (5) zur Aufnahme mindestens eines Samens, einer Zwiebel oder Knolle (2), wobei das erste Fach (5) im Wesentlichen frei von Wasser ist;
ein zweites Fach (7) zur Aufnahme von Nährstoffen (9), welche das Wachstum des Samens, der Zwiebel oder Knolle (2) ermöglichen, wobei das zweite Fach (7) unter dem ersten Fach (5) angeordnet ist, und
ein Bodenteil (13), wobei der Behälter (1) weiter umfasst
eine Trennschicht (11), die das erste und das zweite Fach (5, 7) voneinander trennt, und wobei das Bodenteil (13) des Gehäuses (3) eine abgerundete Form aufweist, **dadurch gekennzeichnet, dass** der Behälter (1) ein Schwerkraftzentrum hat, das so angeordnet ist, dass der Behälter dazu eingerichtet ist, sich selbst aufzurichten, wenn er auf geneigte Weise auf einer Auflagefläche abgelegt wird.

2. Behälter nach Anspruch 1, wobei das zweite Fach (7) eine Nährstoffmatrix (9) umfasst, die Wasser umfasst, wobei das Wasser bevorzugt in der Nährstoffmatrix (9) gebunden ist und/oder wobei die Nährstoffmatrix (9) bevorzugt ein Gel zur Lagerung von Wasser ist.

3. Behälter nach Anspruch 1 oder 2, wobei das Gehäuse (3) aus nicht wasserlöslichen, biologisch abbaubaren Materialien besteht und/oder Teile mit verringerter Wanddicke aufweist.

4. Behälter nach einem der vorhergehenden Ansprüche, wobei das Gehäuse (3) durch aus dem Samen, der Zwiebel oder Knolle (2) hervorgehende Wurzeln durchdringbar ist und wobei das Gehäuse (3) bevorzugt eines oder mehrere der folgenden Materialien umfasst: Papier, Karton, Kunststoffe, Metallplatten oder Geflechte aus diesen Materialien.

5. Behälter nach einem der vorhergehenden Ansprüche, wobei der Behälter (1) Aktivierungsmittel (17) zur Zerstörung der Trennschicht (11) umfasst.

6. Behälter nach Anspruch 5, wobei das Aktivierungsmittel (17) Mittel zum mechanischen Durchdringen und/oder Zerbrechen (17) der Trennschicht (11) umfasst, oder Mittel zum chemischen Auflösen der Trennschicht (11), oder wobei die Trennschicht (11) aus einem im Wesentlichen wasserfreien Material hergestellt ist, das über einer definierten Temperatur schmilzt.

7. Behälter nach einem der vorhergehenden Ansprüche, wobei die Trennschicht (11) eine bzw. ein, bevorzugt spröde, Platte, Gitter, Geflecht oder Folie ist.

8. Behälter nach einem der vorhergehenden Ansprüche, wobei das Gehäuse (3) mindestens wasserdicht in Bezug auf die Umgebung des Behälters (1) in einem Teil des zweiten Fachs (7) ist, oder wobei das Gehäuse (3) wasserdicht in Bezug auf die den Behälter (1) umgebende Umgebung ist.

9. Behälter nach einem der vorhergehenden Ansprüche, wobei das Gehäuse (3) mindestens eine Öffnung (20) in einem oberen Bereich (14) des Gehäuses (3) aufweist, wobei die Öffnung (20) durch eine bevorzugt wasserdichte Abdeckschicht (21) abgedeckt ist.

10. Behälter nach Anspruch 9, wobei die Abdeckschicht (21) eine mindestens teilweise durchsichtige Folie zur Ermöglichung eines Treibhauseffekts in dem Gehäuse (3) ist, wobei die Abdeckschicht (21) bevorzugt für eine aus dem Samen, der Zwiebel oder Knolle (2) hervorgehende Pflanze durchdringbar ist.

11. Behälter nach Anspruch 9 oder 10, wobei das erste Fach (5) eine zusätzliche Abdeckschicht (23) umfasst, die zwischen der die Öffnung (20) bedeckenden Abdeckschicht (21) und der Trennschicht (11) angeordnet ist, sodass ein erster Raum zwischen der zusätzlichen Abdeckschicht (23) und der Trennschicht (11) bereitgestellt wird und ein zweiter Raum zwischen der zusätzlichen Abdeckschicht (23) und der die Öffnung bedeckenden Abdeckschicht (21) bereitgestellt wird.

12. Behälter nach Anspruch 11, wobei eine der Abdeckschichten (21, 23), bevorzugt die zusätzliche Abdeckschicht (23), im Wesentlichen opak ist.

13. Behälter nach einem der Ansprüche 9 bis 12, wobei mindestens eine der Abdeckschichten (21, 23) oder das Gehäuse (3) einen Schlitz (25) oder ein Loch (27) zum Einbringen eines Samens, einer Zwiebel oder Knolle (2) in das erste Fach (5) umfasst; oder wobei das erste Fach (5) einen Samen, eine Zwiebel oder Knolle (2) umfasst.

14. Behälter nach einem der vorhergehenden Ansprüche, wobei die Trennschicht (11) Nahrung für den Samen, die Zwiebel oder Knolle (2) umfasst, bevorzugt in Form eines anorganischen Salzes, spezieller Hydroxyapatit, Ca₃(PO₄)₂ oder KCl.

15. Verfahren zur Verwendung des Behälters (1) nach einem der vorhergehenden Ansprüche, wobei das Verfahren die aufeinanderfolgenden Schritte umfasst des:
a) Lagerns des Behälters (1);
b) Aktivierens des Behälters (1) durch Zerstören der Trennschicht (11); und
c) Ablegens des Behälters (1) auf einer zum Pflanzen des Samens, der Zwiebel oder Knolle (2) gedachten Oberfläche.

## Revendications

1. Récipient (1) pour l'entreposage et pour la plantation de semences, de bulbes ou de tubercules (2), le récipient (1) comprenant :
un logement (3) constitué d'une matière non soluble dans l'eau, le logement comprenant :
un premier compartiment (5) destiné à recevoir au moins une semence, un bulbe ou un tubercule (2), le premier compartiment (5) étant essentiellement exempt d'eau ;
un deuxième compartiment (7) destiné à recevoir des éléments nutritifs (9) qui permettent de faire croître la semence, le bulbe ou le tubercule (2), le deuxième compartiment (7) étant disposé en dessous du premier compartiment (5) ; et
une portion de base (13) ;
dans lequel le récipient (1) comprend en outre une couche de séparation (11) qui sépare le premier et le deuxième compartiment (5, 7), et dans lequel la portion de base (13) du logement (3) possède une configuration de forme arrondie ;
**caractérisé en ce que** le récipient (1) possède un centre de gravité disposé de manière telle que le récipient (1) est conçu pour se redresser de lui-même lorsqu'il est déposé sur une surface de support par basculement.

2. Récipient selon la revendication 1, dans lequel le deuxième compartiment (7) comprend une matrice (9) faisant office de substance nutritive comprenant de l'eau, l'eau étant de préférence liée dans la matrice (9) faisant office de substance nutritive et/ou dans lequel la matrice (9) faisant office de substance nutritive est de préférence un gel pour le stocker l'eau.

3. Récipient selon la revendication 1 ou 2, dans lequel le logement (3) est constitué par des matières biodégradables non solubles dans l'eau et/ou possède des portions dont l'épaisseur de paroi est réduite.

4. Récipient selon l'une quelconque des revendications précédentes, dans lequel des racines qui émanent de la semence, du bulbe ou du tubercule (2) peuvent pénétrer dans le logement (3), et dans lequel le logement (3) comprend de préférence une ou plusieurs matières choisies parmi celles indiquées ci-après : du papier, du carton, une matière plastique, des feuilles de métal ou des tresses de ces matières.

5. Récipient selon l'une quelconque des revendications précédentes, dans lequel le récipient (1) comprend un moyen d'activation (17) pour la destruction de la couche de séparation (11).

6. Récipient selon la revendication 5, dans lequel le moyen d'activation (17) comprend un moyen pour la pénétration et/ou la rupture mécanique (17) de la couche de séparation (11) ou un moyen pour la dissolution chimique de la couche de séparation (11), ou dans lequel la couche de séparation (11) est constituée d'une matière essentiellement exempte d'eau qui entre en fusion au-dessus d'une température définie.

7. Récipient selon l'une quelconque des revendications précédentes, dans lequel la couche de séparation (11) représente une plaque, une grille, une tresse ou un film, de préférence friable.

8. Récipient selon l'une quelconque des revendications précédentes, dans lequel le logement (3) est au moins étanche à l'eau par rapport à l'environnement du récipient (1) dans une portion du deuxième compartiment (7) ou dans lequel le logement (3) est étanche à l'eau par rapport à l'environnement qui entoure le récipient (1).

9. Récipient selon l'une quelconque des revendications précédentes, dans lequel le logement (3) possède au moins un orifice (20) dans une zone supérieure (14) du logement (3), l'orifice (20) étant recouvert d'une couche de revêtement (21) de préférence étanche à l'eau.

10. Récipient selon la revendication 9, dans lequel la couche de revêtement (21) comprend une feuille mince au moins partiellement transparente pour rendre possible un effet de serre au sein du logement (3), la couche de revêtement (21) étant de préférence en mesure d'être traversée par une plante émanant de la semence, du bulbe ou du tubercule (2).

11. Récipient selon la revendication 9 ou 10, dans lequel le premier compartiment (5) comprend une couche de revêtement supplémentaire (23) disposée entre la couche de revêtement (21) qui recouvre l'orifice (20) et la couche de séparation (11), de manière telle qu'un premier espace est ménagé entre la couche de revêtement supplémentaire (23) et la couche de séparation (11) et qu'un deuxième espace est ménagé entre la couche de revêtement supplémentaire (23) et la couche de revêtement (21) qui recouvre l'orifice.

12. Récipient selon la revendication 11, dans lequel une des couches de revêtement (21, 23), de préférence la couche de revêtement supplémentaire (23), est essentiellement opaque.

13. Récipient selon l'une quelconque des revendications 9 à 12, dans lequel au moins un élément choisi parmi les couches de revêtement (21, 23) ou le logement (3) comprend une fente (25) ou un trou (27) pour introduire une semence, un tubercule ou un bulbe (2) dans le premier compartiment (5), ou dans lequel le premier compartiment (5) comprend une semence, un bulbe ou un tubercule (2).

14. Récipient selon l'une quelconque des revendications précédentes, dans lequel la couche de séparation (11) comprend un agent de nutrition pour la semence, le bulbe ou le tubercule (2), de préférence sous la forme d'un sel inorganique, en particulier l'hydroxyapatite, du Ca₃(PO₄)₂ ou du KCl.

15. Procédé d'utilisation du récipient (1) selon l'une quelconque des revendications précédentes, le procédé comprenant les étapes séquentielles consistant à :
a) entreposer le récipient (1) ;
b) activer le récipient (1) par la destruction de la couche de séparation (11) ; et
c) déposer le récipient (1) sur une surface conçue pour la plantation d'une semence, d'un bulbe ou d'un tubercule (2).
